# EUROPÄISCHE PATENTSCHRIFT

(11) **EP 0 739 889 B1**
(45) Veröffentlichungstag und Bekanntmachung des Hinweises auf die Patenterteilung: **08.12.1999**
(21) Anmeldenummer: 96103017.8
(22) Anmeldetag: 29.02.1996
(51) Int. Cl.: C07D 321/00, C07D 323/00

(54) **Verfahren zur kontinuierlichen Herstellung von macrocyclischen Verbindungen**
Process for the continuous production of macrocyclic compounds
Procédé pour la fabrication en continu de composés macrocycliques

(30) Priorität: 29.04.1995 DE 19515888
(43) Veröffentlichungstag der Anmeldung: 30.10.1996
(73) Patentinhaber: Degussa-Hüls Aktiengesellschaft, 60287 Frankfurt am Main (DE)
(72) Erfinder: Köhler, Günther, Dr., 45770 Marl (DE); Feld, Marcel, Dr., 51147 Köln (DE); Metz, Josef, Dr., 45770 Marl (DE)

(56) Entgegenhaltungen:
- EP-A- 0 260 680
- US-A- 4 803 288
- DATABASE WPI Section Ch, Week 8044 Derwent Publications Ltd., London, GB; Class D23, AN 80-77899C XP002009082 & JP-A-55 120 581 (MITSUI PETROCHEM. IND. KK) , 18.September 1980

## Beschreibung

Macrocyclische Verbindungen, wie z. B. macrocyclische Ester, spielen auf Grund ihrer bekannten Moschus- und Ambranote und darüber hinaus als Fixateur in der Riechstoffindustrie eine außerordentlich große Rolle. Das größte Marktvolumen besitzen dabei die 15 bis 18 gliedrigen Lactone, Dilactone oder Oxalactone.

Zu deren Herstellung sind viele Herstellverfahren bekannt, bei denen macrocyclische Kondensate durch Thermolyse im Vakuum bei hohen Temperaturen in Gegenwart verschiedener Metallkatalysatoren hergestellt werden können. So wird die Depolymerisation und Cyclisierung aus den Polykondensaten im allgemeinen bei sehr hohen Temperaturen (200 bis 300 °C) und im Vakuum ausgeführt. Die sich bildenden macrocyclischen Verbindungen besitzen unter diesen Bedingungen einen sehr hohen Dampfdruck, so daß sie destillativ aus dem System entfernt werden können. Durch anschließende fraktionierte Destillation bzw. durch Kristallisation lassen sich die Zielprodukte rein und in Riechstoffqualität herstellen.

Problematisch ist jedoch die Auswahl des Mediums, in dem die Reaktion ausgeführt wird. In der EP-PS 0 260 680 (USP 4 709 058) werden z. B. Olefinpolymere, in JP-AS 55-120 581 Polyester, Polyetherglykole, Polyetherglykolester oder nur Polyglykole, in der DE-PS 32 25 431 Paraffin als hochsiedendes Medium für die Depolymerisation vorgeschlagen. All diese Stoffe haben jedoch den Nachteil, daß sie meist einen hohen Schmelzpunkt aufweisen, der die Handhabung erschwert. Ein gravierender Nachteil ist, daß die verwendeten Polyetherglykole und Polyetherglykolester funktionelle Gruppen besitzen, die in unerwünschter Weise an der Polymerisation teilnehmen, was zu deutlichen Ausbeuteverlusten führt.

Ein Nachteil der Paraffine ist der meist zu niedrige Siedepunkt dieser Sumpfmedien, so daß bei der Depolymerisation unter hohen Temperaturen und im Vakuum zusammen mit dem Zielprodukt das Sumpfmedium überdestillieren kann. Dadurch wird bei der Reindestillation oft eine zusätzliche Destillationsstufe erforderlich.

Eine in der Technik wichtige Forderung ist, daß die Viskosität des Sumpfmediums bei Temperaturen von weniger als 50 °C gering ist, so daß es bei Unterbrechungen oder Beendigung der Reaktion bequem dem Reaktor entnommen werden kann, was nach bekannten Verfahren und herkömmlichen Sumpfmedien kaum möglich ist.

Sumpfmedien wie z. B. Paraffine oder Olefinpolymere sind darüber hinaus für die linearen Poly- bzw. Oligoester wenig geeignete Lösemittel. Sie dispergieren diese vielfach nur, wobei die Teilchen zu Blöcken koagulieren können. Abhilfe erzielt man meist durch weitere Verdünnung, wodurch die Raum-Zeit-Ausbeute deutlich reduziert wird, wie es nach bekannten Verfahren der Fall ist.

Nach üblichen und bekannten Verfahren wird die Cyclisierung durch Polykondensation als unerwünschte Nebenreaktion (intermolekulare Vernetzung) begleitet. Dadurch wird die Viskosität des Systems noch stärker erhöht, wobei die Rührbarkeit und Wärmeleitfähigkeit des Gemisches stark abnimmt. Es kommt dabei oft zu Wandanhaftungen und Zersetzungserscheinungen, die Nebengeruchbildung zur Folge haben. Bei der anschließenden Reindestillation sind diese Spuren von Geruchsverunreinigungen nicht oder nur mit sehr hohen Verlusten zu entfernen.

Aufgabe der Erfindung war es nun, ein Herstellverfahren für macrocyclische Verbindungen zu entwickeln, das diese Nachteile nicht aufweist.

Gegenstand der vorliegenden Erfindung ist ein Verfahren zur Herstellung von macrocyclischen Verbindungen, erhältlich durch Kondensation von mindestens difunktionellen Verbindungen und anschließender thermischer Depolymerisation in Gegenwart von Katalysatoren, dadurch gekennzeichnet, daß die thermische Depolymerisation in einem Lösemittel der allgemeinen Formel I 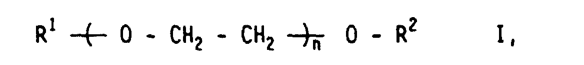 wobei R¹ und R² gleiche oder verschiedene aliphatische Kohlenwasserstoffreste mit 1 bis 6 Kohlenstoffatomen mit gegebenenfalls funktionellen Gruppen darstellen, mit Molekulargewichten von 500 bis 3 000, wodurch sich zwangsläufig der n-Wert ergibt, bei einem Druck von weniger als 50 hPa und bei einer Temperatur von 200 bis 300 °C durchgeführt wird, wobei pro Gewichtsanteil der macrocyclischen Verbindung 5 bis 1 000 Gewichtsteile Lösemittel zur Anwendung kommen.

Als hochsiedendes Medium für die thermische Depolymerisation kommen Polyethylenglykoldialkylether zur Anwendung, mit Molekulargewichten zwischen 500 bis 3 000, vorzugsweise jedoch zwischen 1 000 bis 2 000, wobei die endständigen OH-Gruppen der Polyethylenglykoldialkylether mit Alkylgruppen verethert sind.

Die das Polyglykol verethernden Alkylgruppen R¹ und R² können z. B. Methyl-, Ethyl-, iso-Propyl-n-Propyl-, n-Butyl- oder sec. Butylgruppen sein. Es können Gemische verschiedener Polyethylenglykoldialkylether, sowie mit unterschiedlichen Alkylgruppen verethernde Polyethylenglykoldialkylether, verwendet werden.

Es war überraschend, daß die erfindungsgemäß verwendeten Lösemittel unter den Reaktionsbedingungen der Depolymerisation in Gegenwart von Lewis-Säuren stabil sind. Dies konnte man nicht erwarten, da sich bekanntlich herkömmliche Ether in Gegenwart von Lewis-Säure bei hohen Temperaturen spalten.

Es ist wirkungsvoll, wenn auf 1 Gewichtsteil des Oligomers wenigstens 5 bis 1 000 Gewichtsteile Polyethylenglykoldialkylether verwendet werden.

Bei der Depolymerisation und Cyclisierung der linearen Oligomeren werden übliche und nach dem Stand der Technik bekannte Katalysatoren eingesetzt, wie z. B. Alkalimetalle und deren Salze, Magnesium-, Mangan-, Cadmium-, Eisen-, Cobalt-, Zinn-, Blei-, Aluminium- und Ti-Salze. Die Menge des Katalysators liegt je nach Verwendung des entsprechenden Typs zwischen 0,1 bis 20 Gew.-%, vorzugsweise jedoch zwischen 0,5 und 10 Gew.-%, bezogen auf 100 Gew.-% Oligomer.

Die Durchführung des Verfahrens wird zunächst mit der Kondensation eingeleitet, was nach bekannten Verfahren bei erhöhten Temperaturen mit oder ohne Katalysator erfolgen kann. Dabei wird die α,β-Hydroxycarbonsäure oder α,β-Dicarbonsäure oder Ester derselben mit einem Glykol umgesetzt. Das dabei entstehende Wasser oder der Alkohol wird abdestilliert oder mit Hilfe eines Schleppmittels bzw. unter Zuhilfenahme von geringem Vakuum entfernt.

Danach wird das Oligomer kontinuierlich in den Reaktor überführt, in dem das hochsiedende Medium zusammen mit der Katalysatorkomponente vorgelegt sind. Bei hohen Temperaturen von 200 bis 300 °C, bevorzugt 220 bis 265 °C, und einem Vakuum von weniger als 50 hPa findet nun die Depolymerisation und Cyclisierung statt. Das Zielprodukt destilliert unter den vorgenannten Bedingungen über, wobei das aus der Depolymerisation stammende Glykol (z. B. Ethylenglykol) oder auch ein bewußter Überschuß des Glykols die cyclische Komponente mitschleppt.

Nach Phasentrennung kann das separierte Glykol der Kondensation am Beginn der Reaktion wieder zugeführt werden. Diese Zurückführung ist darüber hinaus auch deshalb vorteilhaft, da geringe Mengen von gelöster macrocyclischer Verbindung dem Stoffkreislauf in dieser Weise nicht verloren gehen.

Nach diesem Verfahren können viele macrocyclische Verbindungen, wie z. B. Ester, Lactone, Lactame, Etherlactone, Dilactone und Etherdilactone hergestellt werden.

Es ist demnach besonders geeignet zur Herstellung von macrocyclischen Estern und Lactonen mit 6 bis 20, bevorzugt 8 bis 15, Kohlenstoffatomen, da sie sich durch das erfindungsgemäße Verfahren besonders rein darstellen lassen, was ihrer Verwendung als Riechstoffe in hohem Maße zugute kommt.

### Beispiel 1

### Herstellung eines linearen Oligoesters

460 Teile Dodecandisäure und 1 380 Teile Ethylenglykol werden bei 160 bis 200 °C unter Rühren und bei Normaldruck ca. 4 h am Rückfluß erhitzt. Danach wird bei geringem Vakuum (ca. 500 hPa) oder einem schwachen Stickstoffstrom das Reaktionswasser destillativ entfernt. Der auf diese Weise erhaltene lineare Oligoester hat einen Erstarrungspunkt von 25 bis 35 °C und einen sehr niedrigen Polymerisationsgrad.

### Herstellung eines cyclischen Esters

200 g dieses Oligoethylendodecandioats (durchschnittliches Molekulargewicht ca. 500) werden innerhalb von 4 h in einen Kolben mit Destillationsaufsatz und Kühler dosiert, der mit ca. 100 Teilen Polyethylenglykoldialkylether (durchschnittliches Molekulargewicht 2 000) bei vermindertem Druck von 5 hPa und einer Temperatur von 260 °C beaufschlagt ist.

Gleichzeitig werden innerhalb der gleichen Zeit 0,5 g des Sn enthaltenden Katalysators als Lösung in einem inerten Lösemittel dosiert. Nun destillieren zusammen mit 147 g Ethylenglykol 53 g des cyclischen Esters der Dodecandisäure über. Die Ausbeute entspricht dabei ca. 93 % der Theorie. Nach Phasentrennung wird die obere Phase des cyclischen Esters feindestilliert, wobei man praktisch ohne Verluste das Dilacton > 99 % in hervorragender Riechstoffqualität erhält. Durch Rückführung der unteren Ethylenglykolphase, in der ein Teil des cyclischen Esters gelöst ist (ca. 5 bis 7 %), hat man kaum Stoffverluste.

Der gleiche Sumpf kann mehr als 50 dieser Zyklen verwendet werden. Danach ist er trotz seiner relativ dunklen Färbung bei 25 bis 35 °C noch fließfähig und kann relativ leicht aus dem Reaktor entfernt werden. Der Reaktor kann leicht mit in der Technik gängigen Lösemitteln wie Toluol oder niederen Alkoholen gespült werden, ohne daß Wandanhaftungen vorhanden sind.

### Beispiel 2

### Herstellung des linearen Oligoesters

816 g (3 mol) Tridecandisäuredimethylester und 1 860 g (30 mol) Ethylenglykol werden unter Rühren bei Normaldruck und 160 °C im Sumpf mit oder ohne einen zur Umesterung üblichen Katalysator (z. B. p-Toluolsulfonsäure) erhitzt. Unter schwachem Vakuum (ca. 600 hPa) oder einem geringen Stickstoffstrom werden ca. 190 g Methanol destillativ entfernt.

### Herstellung des cyclischen Esters

In eine Apparatur mit Destillationsaufsatz und Kühler wurden in einen 1 l Kolben, der mit ca. 250 g Polyethylenglykoldimethylether (Molekulargewicht 2 000) und vermindertem Druck von ca. 10 hPa beaufschlagt und auf 260 °C erhitzt ist, innerhalb von 3 h 185 g des wie oben beschriebenen Oligoethylentridecandioats dosiert. Gleichzeitig werden 0,3 g eines Zinn enthaltenden Katalysators in den Reaktor dosiert. Nun destillieren kontinuierlich ein Gemisch von 56 g Ethylenbrassylat und 140 g Ethylenglykol über, das sich in zwei Phasen trennt. Zur vollständigen Extraktion kann aus der unteren Ethylenglykolphase mit Hilfe eines üblichen Extraktionsmittels Ethylenbrassylat noch vollständig isoliert werden, wobei weitere 5 g gewinnbar sind. Die Glykolphase kann allerdings auch in den Prozeß zur Herstellung des Oligoesters recyclisiert werden, ohne die Extraktion auszuführen. Durch Redestillation im Hochvakuum wurde das rohe Ethylenbrassylat auf eine Reinheit von 99 % gebracht, wobei 58 g mit einer Ausbeute von ca. 95 % d. Th. erhalten wurden.

### Beispiel 3

### Herstellung des Oligomerengemisches

Ein Gemisch aus 387 g (1,5 mol) 15-Hydroxypentadecansäure und 744 g (12 mol) Ethylenglykol werden bei 200 °C Sumpftemperatur 8 h gerührt, wobei ca. 25 g Wasser zusammen mit geringen Mengen Ethylenglykol überdestillieren. Mit Hilfe eines geringen Stickstoffstromes kann das Entfernen des Wassers erleichtert werden. Da so erhaltene Oligomerengemisch wird nun zur Cyclisierung eingesetzt.

### Cyclisierungsstufe

Die Cyclisierungsstufe wird ausgeführt, indem auf einen mit ca. 300 g Polyethylenglykoldimethylether (Molekulargewicht 1 500 - 2 000) beaufschlagten 1 l Kolben mit Destillationsaufsatz und Kühler bei 250 °C und 5 hPa innerhalb von 6 h 300 g des oben beschriebenen Oligomerengemisches zusammen mit 1 g Tetrabutyltitanat dosiert werden. Gleichzeitig destillieren innerhalb von 6 h ein Gemisch von 295 g Cyclopentadecanolid und Ethylenglykol über.

Mit Hilfe von Cylcohexan kann das Gemisch extrahiert werden. Es läßt sich eine obere Cyclohexan/Cyclopentadecanolidphase und eine untere Ethylenglykolphase abtrennen. Nach Entfernen des Cyclohexans aus der oberen Phase und Destillation des Rückstandes im Vakuum werden 91 g Cyclopentadecanolid mit einer Reinheit von 99 % gewonnen, was einer Ausbeute von 93 % entspricht.

## Patentansprüche

1. Verfahren zur Herstellung von macrocyclischen Verbindungen, erhältlich durch Kondensation von mindestens difunktionellen Verbindungen und anschließender thermischer Depolymerisation in Gegenwart von Katalysatoren,
dadurch gekennzeichnet,
daß die thermische Depolymerisation in einem Lösemittel der allgemeinen Formel I 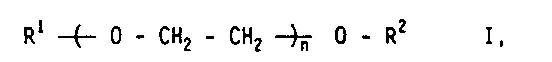 wobei R¹ und R² gleiche oder verschiedene aliphatische Kohlenwasserstoffreste mit 1 bis 6 Kohlenstoffatomen mit gegebenenfalls funktionellen Gruppen darstellen, mit Molekulargewichten von 500 bis 3 000, wodurch sich zwangsläufig der n-Wert ergibt, bei einem Druck von weniger als 50 hPa und bei einer Temperatur von 200 bis 300 °C durchgeführt wird, wobei pro Gewichtsanteil der macrocyclischen Verbindung 5 bis 1 000 Gewichtsteile Lösemittel zur Anwendung kommen.

2. Verfahren nach Anspruch 1,
dadurch gekennzeichnet,
daß die thermische Depolymerisation bei Temperaturen von 220 bis 265 °C durchgeführt wird.

3. Verfahren nach mindestens einem der Ansprüche 1 bis 2,
dadurch gekennzeichnet,
daß Lactone, Lactame, Etherlactone, Dilactone und Etherdilactone erhalten werden.

4. Verfahren nach mindestens einem der Ansprüche 1 bis 3,
dadurch gekennzeichnet,
daß Ester und Lactone mit 6 bis 20 Kohlenstoffatomen erhalten werden.

5. Verfahren nach mindestens einem der Ansprüche 1 bis 4,
dadurch gekennzeichnet,
daß Ester und Lactone mit 8 bis 15 Kohlenstoffatomen erhalten werden.

## Claims

1. A process for the preparation of macrocyclic compounds obtainable by condensation of at least difunctional compounds and subsequent thermal depolymerization in the presence of catalysts,
characterized in that
the thermal depolymerization is carried out in a solvent of the general formula I
R¹ (̵O-CH₂-CH₂)̵ₙO-R² I,
where R¹ and R² are identical or different aliphatic hydrocarbon radicals having from 1 to 6 carbon atoms with or without functional groups, having molecular weights of from 500 to 3000, which automatically gives the n value, at a pressure of less than 50 hPa and at a temperature of from 200 to 300°C, where, per part by weight of the macrocyclic compound, from 5 to 1000 parts by weight of solvent are used.

2. A process according to claim 1,
characterized in that
the thermal depolymerization is carried out at temperatures of from 220 to 265°C.

3. A process according to at least one of claims 1 to 2, characterized in that
lactones, lactams, ether lactones, dilactones and ether dilactones are obtained.

4. A process according to at least one of claims 1 to 3, characterized in that
esters and lactones having from 6 to 20 carbon atoms are obtained.

5. A process according to at least one of claims 1 to 4, characterized in that
esters and lactone having from 8 to 15 carbon atoms are obtained.

## Revendications

1. Procédé de fabrication de composés macrocycliques accessible par condensation de composés au moins difonctionnels et de polymérisation thermique consécutive en présence de catalyseurs,
caractérisé en ce que
la dépolymérisation thermique est effectuée dans un agent dissolvant de formule générale I 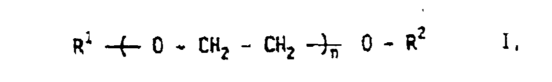 dans laquelle R¹ et R² forment des restes d'hydrocarbures aliphatiques identiques ou différents, ayant de 1 à 6 atomes de carbone avec des groupes éventuellement fonctionnels, ayant des poids moléculaire allant de 500 à 3000, grâce à quoi la valeur n se déduit obligatoirement, à une pression de moins de 50 hPa et une température de 200 à 300°C, dans lequel par fraction en poids de composé macrocyclique, 5 à 1000 parties en poids d'agent dissolvant viennent à utilisation.

2. Procédé selon la revendication 1,
caractérisé en ce que
la dépolymérisation thermique s'effectue à des températures allant de 220 à 265°C.

3. Procédé selon au moins une des revendications 1 à 2,
caractérisé en ce que
des lactones, des lactames, des éthers de lactone, des dilactones et des éthers de dilactone sont obtenus.

4. Procédé selon au moins une des revendications 1 à 3,
caractérisé en ce que
des esters et des lactones ayant de 5 à 20 atomes de carbone sont obtenus.

5. Procédé selon au moins une des revendications 1 à 4,
caractérisé en ce que
des esters et des lactones ayant de 8 à 15 atomes de carbone, sont obtenus.
